# EUROPÄISCHE PATENTSCHRIFT

(11) **EP 0 413 057 B1**
(45) Veröffentlichungstag und Bekanntmachung des Hinweises auf die Patenterteilung: **09.11.1994**
(21) Anmeldenummer: 89116061.6
(22) Anmeldetag: 31.08.1989
(51) Int. Cl.: A61F 2/16

(54) **Intraokulare Hinterkammerlinse**
Posterior chamber intraocular lens
Lentille intraoculaire de chambre postérieure

(30) Priorität: 18.08.1989 DE 3927360
(43) Veröffentlichungstag der Anmeldung: 20.02.1991
(73) Patentinhaber: Chiron Adatomed Pharmazeutische und Medizintechnische Gesellschaft mbH, 85609 Dornach (DE)
(72) Erfinder: Greite, Prof. Dr. Jürgen-H., D-8000 München 90 (DE)
(74) Vertreter: Nöth, Heinz, Dipl.-Phys.

(56) Entgegenhaltungen:
- EP-A- 0 161 765
- EP-A- 0 246 216
- FR-A- 2 581 535
- FR-A- 2 584 919
- US-E- 32 525

## Beschreibung

Die Erfindung betrifft eine intraokulare Hinterkammerlinse nach dem Oberbegriff des Patentanspruchs 1.

Bei einer derartigen aus der EP-A-246 216 bekannten Intraokularlinse sind an den Linsenkörper zwei gebogene längliche Fixationsschlaufen angeformt, die sich nach außen vom Linsenkörper entlang eines Bogens abseits von der Peripherie des Linsenkörpers erstrecken. Die an den Linsenkörper angeformten Anfangsstücke der beiden Fixationsschlaufen sind geradlinig ausgebildet und erstrecken sich längs ihrer Bogenform so weit vom Linsenkörper weg, daß im implantierten Zustand die Linse unter Spannung in den Kapselsack eingesetzt ist.

Aufgabe der Erfindung ist es, eine intraokulare Hinterkammerlinse der eingangs genannten Art zu schaffen, die spannungsfrei im Kapselsack implantiert werden kann und somit den physiologischen Verhältnissen der natürlichen Augenlinse am nächsten kommt.

Diese Aufgabe wird erfindungsgemäß durch die kennzeichnenden Merkmale des Anspruchs 1 gelöst.

In vorteilhafter Weise läßt sich durch die Erfindung eine Linse schaffen, die einen Linsenkörper mit großer Querschnittsfläche hat, der spannungsfrei im Kapselsack fixierbar ist. Insbesondere sind die kreisbogenförmigen Fixationsschlaufen dem Äquator des Kapselsackes, in welchen die Linse implantiert wird, angepaßt. Vor allem ist es bei der erfindungsgemäßen Intraokularlinse möglich, bei der Implantation den Außenumfang der Fixationsschlaufen auf den Umfang des optischen Linsenkörpers, der bevorzugt etwa 7 mm beträgt, zu verringern, so daß man mit einer relativ geringen Abmessung der Öffnung auskommt, die für die Implantation der Linse an der Vorderseite des Kapselsackes vorzusehen ist. Nach dem Einsetzen der Linse in den Kapselsack nehmen die Fixationsschlaufen wieder etwa ihre ursprüngliche Stellung ein und passen sich dabei an den Äquator des Kapselsackes an. Sie liegen dann an ihrem gesamten Umfangsbereich am Äquator des Kapselsackes spannungsfrei an. Ausbeulungen im Äquatorbereich des Kapselsackes sind damit vermieden. Da die Öffnung in der Vorderseite des Kapselsackes relativ klein bemessen sein kann, besteht auch keine Gefahr des Herausfallens der Linse aus dem Kapselsack. Man erreicht trotz radialer Aufspannung der Fixationsschlaufen eine spannungsfreie Implantation der Linse im Kapselsack, da der Außenumfang der Fixationsschlaufen sich an den Äquator des Kapselsackes anpaßt.

Anhand der beigefügten Figuren wird an einem Ausführungsbeispiel die Erfindung noch näher erläutert. Es zeigt:
- Fig. 1: eine Draufsicht auf ein Ausführungsbeispiel der Erfindung; und
- Fig. 2: eine Seitenansicht des in Fig. 1 dargestellten Ausführungsbeispiels.

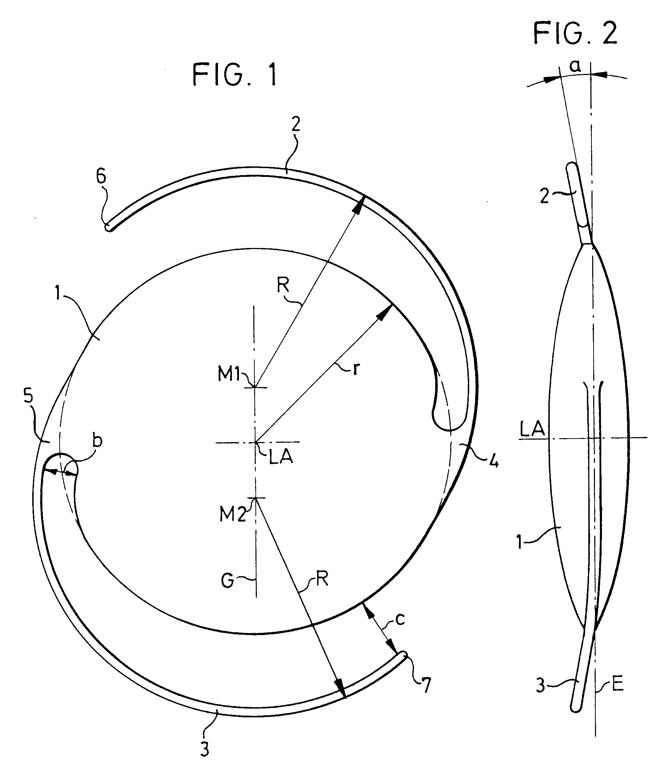

Das in den Figuren 1 und 2 dargestellte Ausführungsbeispiel einer intraokularen Hinterkammerlinse besitzt einen kreisrunden Linsenkörper 1 als optischen Teil. Beim dargestellten Ausführungsbeispiel ist der Linsenkörper 1 bikonvex ausgebildet und besitzt eine relativ große Dicke, die der eines Scheitelbrechwerts in der Größenordnung von 25,00 m⁻¹ (dpt) entspricht. Es ist natürlich auch möglich, den Linsenkörper 1 für geringere Scheitelbrechwerte, beispielsweise von 10,0 m⁻¹ (dpt), dünner auszubilden. Der Durchmesser des Linsenkörpers 1 beträgt bevorzugt 7,0 mm.

Als Linsenkörpermaterial eignet sich jedes augenverträgliche Material. Bevorzugt kommt hochpolymeres Polymethylmethacrylat zum Einsatz. Das Linsenkörpermaterial kann z. B. durch Einpolymerisieren mit einem UV-Absorber versehen sein.

In diametral am Linsenkörperumfang liegenden Anformungsstellen 4, 5 sind tangential zwei Fixationsschlaufen 2 und 3 an den Linsenkörper 1 angeformt. Diese Anformungsstellen 4 und 5, welche Erweiterungen gegenüber der Kreisform (strichlierte Linien in Fig. 1) des Linsenkörpers 1 darstellen, liegen symmetrisch zu einer Geraden G, welche durch eine Linsenkörperachse LA und zwei Mittelpunkte M1 und M2 verläuft. Die Mittelpunkte M1 und M2 sind Mittelpunkte von Kreisbögen, entlang welcher sich die beiden Fixationsschlaufen 2 und 3 erstrecken. Die äußeren Umfänge dieser Kreisbögen der Fixationsschlaufen 2 und 3 liegen jeweils auf Kreisen mit gleichem Radius R (ca. 4 mm) um die Mittelpunkte M1 bzw. M2. Die beiden Mittelpunkte M1 und M2 besitzen von der Linsenkörperachse LA gleiche Abstände (ca. 1 mm). Der Umfang des Linsenkörpers 1 liegt auf einem Kreis um die Linsenkörperachse LA mit einem Radius r (beim dargestellten Ausführungsbeispiel r = 3,5 mm). Die beiden in Bezug auf die Linsenkörperachse LA äußersten Kreibogensegmente der Fixationsschlaufen 2 und 3 liegen auf einem Kreis um einen auf der Linsenkörperachse LA liegenden Mittelpunkt, der einen Durchmesser von etwa 9,5 mm bis 11,5 mm aufweist. Bevorzugt ist dieser Durchmesser mit 10,0 mm bemessen. Die Fädchendicke beträgt ca. 1,5 mm beim Ausführungsbeispiel.

Der Abstand, den die jeweilige Fixationsschlaufe 2 bzw. 3 im Bereich der Anformungsstelle 4 bzw. 5 gegenüber dem dort vorhandenen Umfangsteil des Linsenkörpers 1 aufweist, ist kürzer bemessen als der Abstand, den die freien Enden 6 bzw. 7 der Fixationsschlaufen 2 bzw. 3 gegenüber dem dortigen Umfangsbereich des Linsenkörpers 1 haben. Der Abstand b der jeweiligen Fixationsschlaufe im Bereich der Anformungsstelle 4 bzw. 5 beträgt beim Ausführungsbeispiel ca. 0,4 mm und der Abstand c im Bereich des freien Endes 6 bzw. 7 beträgt beim Ausführungsbeispiel ca. 1,1 mm.

Wie aus der Fig. 1 ferner zu ersehen ist, weicht der Linsenkörper 1 im Bereich der Anformungsstellen 4 und 5 etwas von der Kreisform ab und besitzt hier gering bemessene Ausnehmungen, die die optische Funktion des Linsenkörpers jedoch nicht beeinträchtigen. Es wird auf diese Weise auch gewährleistet, daß in Zusammenwirkung mit der flexiblen Anformung der beiden Fixationsschlaufen 2 und 3 beim Implantieren der Linse in den Kapselsack die beiden Fixationsschlaufen 2 und 3 bis auf den Durchmesser des Linsenkörpers 1 in Richtung zur Linsenkörperachse LA hin gebogen werden können, so daß sich der Gesamtdurchmesser der Intraokularlinse bei der Implantation wesentlich verringern läßt. Die Fixationsschlaufen 2 und 3 erweitern sich nach dem Einsetzen der Linse in den Kapselsack und liegen am Äquatorbereich des Kapselsackes ohne Spannung an, da sie an den Äquatorbereich des Kapselsackes an ihren Außenumfangen angepaßt sind.

Wie die Fig. 2 zeigt, sind die Fixationsschlaufen 2 und 3 gegenüber einer Ebene E, in welcher der kreisförmige Umfang des Linsenkörpers 1 liegt, um einen spitzen Winkel nach vorne, d. h. im implantierten Zustand in Richtung zur Hornhaut des Auges hin, abgewinkelt. Dieser spitze Winkel a beträgt bevorzugt 10°. Auch hierdurch wird eine einwandfreie Fixation, die insbesondere gegen Herausrutschen nach vorne gesichert ist, gewährleistet.

## Patentansprüche

1. Intraokulare Hinterkammerlinse für eine Implantation im menschlichen Auge mit einem optischen Linsenkörper und einer einstückig mit dem Linsenkörper ausgebildeten Haptik in Form von zwei elastisch verformbaren Fixationsschlaufen, die an zur Linsenachse diametral liegenden Anformungsstellen tangential am Umfang des Linsenkörpers mit bis zu ihren freien Enden hin sich vergrößerndem Abstand vom Umfang des Linsenkörpers angeformt sind und in gleicher Umfangsrichtung sich erstrecken,
**gekennzeichnet,**
durch die Kombination, daß die Außenumfänge der beiden Fixationsschlaufen (2, 3), beginnend von der jeweiligen Anformungsstelle (4, 5), welche als Erweiterung gegenüber der Kreisform des Linsenkörpers (1) ausgebildet ist, bis zum jeweiligen freien Ende (6, 7) der Fixationsschlaufe (2, 3) sich auf Kreisbögen um Mittelpunkte (M1, M2) erstrecken, die diametral zur Linsenkörperachse (LA) liegen und von der Linsenkörperachse (LA) gleichen Abstand haben, und daß in Bezug auf die Linsenkörperachse (LA) die äußersten Kreisbogensegmente der Fixationsschlaufen (2, 3) auf einem Kreis mit einem Durchmesser von 9,5 mm bis 11,5 mm um die Linsenkörperachse (LA) als Mittelpunkt liegen.

2. Intraokulare Hinterkammerlinse nach Anspruch 1, dadurch gekennzeichnet, daß die beiden Fixationsschlaufen (2, 3) bezüglich einer Ebene (E), in welcher der Linsenumfang liegt, nach vorne einen spitzen Winkel (a) aufweisen.

3. Intraokulare Hinterkammerlinse nach Anspruch 2, dadurch gekennzeichnet, daß der spitze Winkel 10° beträgt.

4. Intraokulare Hinterkammerlinse nach einem der Ansprüche 1 bis 3, dadurch gekennzeichnet, daß der Abstand der äußersten diametral zueinander liegenden Umfangspunkte der Fixationsschlaufen voneinander 9,5 bis 10,0 mm beträgt.

5. Intraokulare Hinterkammerlinse nach einem der Ansprüche 1 bis 4, dadurch gekennzeichnet, daß der Durchmesser des Linsenkörpers (1) etwa 7,0 mm beträgt.

6. Intraokulare Hinterkammerlinse nach einem der Ansprüche 1 bis 5, dadurch gekennzeichnet, daß das Linsenkörpermaterial hochpolymeres Polymethylmethacrylat ist.

7. Intraokulare Hinterkammerlinse nach einem der Ansprüche 1 bis 6, dadurch gekennzeichnet, daß das Linsenmaterial einen UV-Absorber aufweist.

8. Intraokulare Hinterkammerlinse nach einem der Ansprüche 1 bis 7, dadurch gekennzeichnet, daß der Linsenkörper (1) bikonvex ausgebildet ist.

9. Intraokulare Hinterkammerlinse nach einem der Ansprüche 1 bis 8, dadurch gekennzeichnet, daß die Fixationsschlaufen in der Weise verformbar sind, daß ihr Gesamtumfang auf einem Kreis mit einem Durchmesser von etwa 10 mm liegt.

## Claims

1. An intraocular posterior chamber lens for implantation in the human eye comprising an optical lens body and a haptic which is provided integrally with the lens body in the form of two elastically deformable fixing loops which are formed on the lens body at attachment locations disposed diametrally relative to the lens axis, tangentially to the periphery of the lens body at a spacing from the periphery of the lens body, which increases towards their free ends, and which extend in the same peripheral direction,
characterised by the combination that the outer peripheries of the two fixing loops (2, 3), beginning from the respective attachment location (4, 5) which is in the form of an enlargement relative to the circular shape of the lens body (1), extend to the respective free end (6, 7) of the fixing loop (2, 3) along circular arcs around centre points (M1, M2) which are disposed diametrally relative to the lens body axis (LA) and are at the same spacing from the lens body axis (LA), and that in relation to the lens body axis (LA) the outermost circular arc segments of the fixing loops (2, 3) lie on a circle of a diameter of 9.5 mm to 11.5 mm about the lens body axis (LA) as a centre point.

2. An intraocular posterior chamber lens according to claim 1 characterised in that the two fixing loops (2, 3) are at an acute angle (a) in a forward direction relative to a plane (E) in which the periphery of the lens is disposed.

3. An intraocular posterior chamber lens according to claim 2 characterised in that the acute angle is 10°.

4. An intraocular posterior chamber lens according to one of claims 1 to 3 characterised in that the spacing of the outermost peripheral points, which are disposed diametrally relative to each other, of the fixing loops from each other is from 9.5 to 10.0 mm.

5. An intraocular posterior chamber lens according to one of claims 1 to 4 characterised in that the diameter of the lens body (1) is about 7.0 mm.

6. An intraocular posterior chamber lens according to one of claims 1 to 5 characterised in that the lens body material is high-polymer polymethylmethacrylate.

7. An intraocular posterior chamber lens according to one of claims 1 to 6 characterised in that the lens material has an UV-absorber.

8. An intraocular posterior chamber lens according to one of claims 1 to 7 characterised in that the lens body (1) is of a biconvex configuration.

9. An intraocular posterior chamber lens according to one of claims 1 to 8 characterised in that the fixing loops are deformable in such a way that their overall periphery lies on a circle of a diameter of about 10 mm.

## Revendications

1. Implant intraoculaire pour une implantation dans l'oeil humain, avec un corps de lentille optique et une haptique réalisée d'une seule pièce avec ce dernier, en forme de deux boucles de fixation déformables élastiquement, conformées, en des points diamétraux par rapport à l'axe de la lentille,, à la tangente du pourtour du corps de lentille, avec une distance croissante, par rapport au pourtour du corps précité, jusqu'à leurs extrémités libres, et s'étendant dans le même sens périphérique, caractérisé par la combinaison que les pourtours externes des deux bouc les de fixation (2, 3), à partir du point de conformation respectif (4, 5), réalisé sous forme d'élargissement par rapport à la forme circulaire du corps de lentille (1), s'étendent jusqu'à l'extrémité libre respective (6, 7) de la boucle de fixation (2, 3) sur des arcs de cercle, autour de centres (M1, M2), diamétraux par rapport à l'axe (LA) du corps de lentille et présentant la même distance par rapport à l'axe précité (LA), et que les segments d'arcs de cercle des boucles de fixation (2, 3), extrêmes par rapport à l'axe (LA) du corps de lentille, se situent sur un cercle de 9,5 mm à 11,5 mm de diamètre autour de l'axe (LA) en tant que centre.

2. Implant intraoculaire suivant la revendication 1, caractérisé en ce que les deux boucles de fixation (2, 3) présentent un angle aigu (a) vers l'avant par rapport à un plan (E), dans lequel se situe le pourtour de la lentille.

3. Implant intraoculaire suivant la revendication 2, caractérisé en ce que l'angle aigu est de 10°.

4. Implant intraoculaire suivant l'une quelconque des revendications 1 à 3, caractérisé en ce que la distance mutuel le des points périphériques extrêmes diamétraux des boucles de fixation est de 9,5 à 10,00 mm.

5. Implant intraoculaire suivant l'une quelconque des revendications 1 à 4, caractérisé en ce que le diamètre du corps de lentille (1) est de l'ordre de 7,0 mm.

6. Implant intraoculaire suivant l'une quelconque des revendications 1 à 5, caractérisé en ce que la matière du corps de lentille est un polyméthacrylate de méthyle haut polymère.

7. Implant intraoculaire suivant l'une quelconque des revendications 1 à 6, caractérisé en ce que la matière de la lentille présente un absorbeur UV.

8. Implant intraoculaire suivant l'une quelconque des revendications 1 à 7, caractérisé en ce que le corps de lentille (1) a une réalisation biconvexe.

9. Implant intraoculaire suivant l'une quelconque des revendications 1 à 8, caractérisé en ce que les boucles de fixation sont déformables, de manière que la totalité de leur pourtour se situe sur un cercle de 10 mm de diamètre environ.
